# EUROPEAN PATENT SPECIFICATION

(11) **EP 2 217 221 B1**
(45) Date of publication and mention of the grant of the patent: **27.06.2018**
(21) Application number: 08838580.2
(22) Date of filing: 17.10.2008
(51) Int. Cl.: A61K 9/51

(54) **LDL-LIKE CATIONIC NANOPARTICLES FOR DELIVERYING NUCLEIC ACID GENE, METHOD FOR PREPARING THEREOF AND METHOD FOR DELIVERYING NUCLEIC ACID GENE USING THE SAME**
LDL-ARTIGE KATIONISCHE NANOTEILCHEN ZUR ABGABE EINES NUKLEINSÄUREGENS, VERFAHREN ZU IHRER HERSTELLUNG UND VERFAHREN ZUR ABGABE DES NUKLEINSÄUREGENS DAMIT
NANOPARTICULES CATIONIQUES DU TYPE LDL DESTINÉES À DÉLIVRER UN GÈNE D'ACIDE NUCLÉIQUE, PROCÉDÉ DE PRÉPARATION DE CELLES-CI ET PROCÉDÉ DESTINÉ À DÉLIVRER CE GÈNE D'ACIDE NUCLÉIQUE AU MOYEN DE CES NANOPARTICULES

(30) Priority: 17.10.2007 KR 20070104575
(43) Date of publication of application: 18.08.2010
(73) Proprietor: Korea Advanced Institute of Science and Technology, Taejeon 305-701 (KR); Samyang Corporation, Jongro-gu, Seoul 110-470 (KR)
(72) Inventor: PARK, Tae-Gwan, Daejeon 305-701 (KR); KIM, Hyun-Ryoung, Hwaseong-si Gyeonggi-do 445-787 (KR); KIM, In-Kyoung, Goyang-si Gyeonggi-do 412-511 (KR)
(74) Representative: Patentwerk B.V.
(86) International application number: PCT/KR2008/006167
(87) International publication number: WO 2009/051451

(56) References cited:
- WO-A1-92/21330
- WO-A2-2004/050062
- JP-A- 2006 111 591
- KR-A- 20020 022 868
- US-A- 5 283 185
- US-A1- 2008 020 058
- US-B2- 6 610 321
- HAYES M.E. ET AL.: 'Assembly of nucleic acid-lipid nanoparticles from aqueous-organic monophases.' BIOCHIMICA ET BIOPHYSICA ACTA vol. 1758, 2006, pages 429 - 442, XP005459936

## Description

The present invention relates to a complex of a nucleic acid and a low density lipoprotein (LDL)-like cationic nanoparticle for delivering nucleic acids and a method for preparation thereof and, more particularly, a LDL-like cationic nanoparticle for delivering nucleic acids, which is surface modified and/or re-constructed by lipid components constitutional ingredients of a natural LDL so that it has improved transfection efficiency and stability, a method for preparation of the same, and a method for delivering nucleic acids using the same.

It has been reported that duplexes of synthesized small interfering ribonucleic acids(siRNAs) with 21 to 25 bp length as a regulator of RNA interference may trigger the cleavage of a target messenger RNAs in mammalian cells, which in turn, inhibit expression of specific genes (A. Hamilton, D. Baulcombe, A species of small antisense RNA in post-transcriptional gene silencing in plants, Science 286 (1999) 950-2.; S. Elbashir, J . Harborth, W. Lendeckel, A. Yalcin, K. Weber, T. Tuschi. Duplexes of 21-nucleotide RNAs mediate RNA interference in cultured mammalian cells Nature 411 (2001) 494-8.). Since then, a siRNA with performance of selectively knockingdown target genes in an mRNA level has increased attention in regard to medical treatment of acquired or congenital diseases or disorders by gene therapy.

A siRNA is well known as a desirable drug candidate for gene therapy, however, has a limitation in practical remedy applications due to intracellular and extra-cellular barriers. Negatively charged siRNA shows extremely low cellular uptake and transfection efficiency. A primarily extra-cellular obstacle is chemical degradation by serum nucleases, and therefore, instability of the siRNA in blood causes a problem in intravenous (IV) administration.

In order to overcome the obstacle described above, cationic lipids (De Paula D, Bentley MV, Mahato RI. Hydrophobization and bioconjugation for enhanced siRNA delivery and targeting, RNA. 13 (2007) 431-56) and/or cationc polymers (DJ. Gary, N.Puri, YY. Won, Polymer-based siRNA delivery: Perspectives on the fundamental and phenomenological distinctions from polymer-based DNA delivery, J Control Release. 2007 May 26, [Epub ahead of print]) have been applied to a siRNA delivery system by polyelectrolyte complex formation based on charge complementary activity.

Especially, polycationic polyethyleneimine (PEI) which is widely used in a polyplex formulation to prevent a serum nuclease, may be adhered to a plasma membrane, and thus, be uptaken by an endocytose.

However, it is known that a PEI usually triggers cell death in a variety of cell lines by necrosis or apoptosis and such cytotoxic activity becomes significant with increased molecular weight and/or branching degree of the PEL.

It was shown that a low molecular weight of PEI or a polyethylene glycol (PEG)-grafted PEI copolymer has relatively low cytotoxicity. However, since the cationic polymer has a low amine density and exhibits a low degree of complex condensation, this polymer is not actively transfected in a cell and, as a result, enzymatic(hydrolysis) reaction in a medium and instability of siRNA become increased.

It should be noted that PEG conjugated siRNA and PEI (with a molecular weight of 25K)-based poly-electrolyte complex (PEC) micelles were shown to have improved stability against attack of an enzyme and excellent efficiency of silencing gene, compared to a siRNA/PEI complex.

A non-synthesized carrier derived from natural resources may alleviate the cytotoxicity and, at the same time, may enhance bio-compatible and biodegradable properties, thus preferably being used to deliver siRNAs. A preferred embodiment of the natural carriers may comprise lipid moieties of LDL neither triggering an immune reaction nor being recognized by a reticuloendothelial system (RES). The LDL normally participates in movement of lipids and proteins, in particular, in delivery of cholesterol to external liver tissues throughout systemic circulation thereof.

In fact, a non-hydrophilic drug such as cyclosporine A and amphotericin B lipid complex (ABLC) is combined with LDL particles so as to be efficiently delivered in pre-clinical or clinical therapy. The LDL may be combined with stearyl-poly(L-lysine)(stearyl-PLL), so-called Terplex DNA system, so as to be used in gene delivery (DG. Affleck, L. Yu, DA. Bull, SH. Bailey, SW. Km, Augmentation of myocardial transfection using Terplex DNA: a novel gene delivery system, Gene Ther. 8(5) (2001) 349-53). For this formulation, hydrophobic interaction may occur between PLL ingredients which have interacted with negatively charged DNA.

Meanwhile, it is known that a process of isolating a natural LDL from blood is very difficult and consumes considerable time. For this reason, a reconstituted LDL-like microemulsion (LDE) as a LDL mimic model has been developed using cholesterol ester and phospholipids without incorporating apolipoprotein. From animal studies and clinical therapy, it was disclosed that a LDE introduced into a blood flow acts like a natural LDL (RC. Maranhao, B. Garicochea, EL. Silva, P. Dorlhiac-Llacer, SM. Cadena, D. Coelho, JC. Meneghetti, FJ. Pileggi, DA. Chamone. Plasma kinetics and biodistribution of a lipid emulsion resembling low-density lipoprotein in patients with acute leukemia, Cancer Res. 54 (17) (1994) 4660-6).

However, in order to establish practical applications of approaches based on nucleic acids such as siRNAs, there is still a strong requirement for novel technical solutions and/or strategies to overcome conventional problems such as inferior transfection efficiency and/or stability.

Accordingly, the present invention is directed to solve the problems described above in regard to conventional methods and an object of the present invention is to provide a LDL-like cationic nanoparticle with improved transfection efficiency and stability for delivering a nucleic acid, which is surface modified and/or re-constructed by mimicking components of a natural LDL.

Another object of the present invention is to provide a method for preparation of a LDL-like cationic nanoparticle with improved transfection efficiency and stability for delivering a nucleic acid.

Still yet another object of the present invention is to provide a method for delivering a nucleic acid using the LDL-like cationic nanoparticle with improved transfection efficiency and stability for delivering the nucleic acid, as described above.

In order to accomplish the above objects, a first aspect of the present invention is to provide a complex of a nucleic acid and an LDL-like cationic nanoparticle for delivering a nucleic acid comprising: a lipid core part containing cholesteryl ester and triglyceride; and a cationic surface lipid part containing cholesterol, phospholipids and a cationic lipid, which forms a cationic surface of the lipid core part via hydrophobic interaction, wherein the cationic lipid is combined with the nucleic acid via electrostatic interaction.

The present invention also provides a method for preparation of a LDL-like cationic nanoparticle for delivering a nucleic acid.

Since a LDL-like cationic nanoparticle for delivering a nucleic acid according to the present invention is surface modified and re-constructed by mimicking components of a natural LDL, the inventive nanoparticle exhibits excellent transfection efficiency and stability, thereby effectively delivering nucleic acids, especially, siRNAs to target cells.

### BRIEF DESCRIPTION OF THE DRAWINGS

The above objects, features and advantages of the present invention will become more apparent to those skilled in the related art in conjunction with the accompanying drawings. In the drawings:
Fig. 1 is a schematic view illustrating the assembly of lipid parts of LDL, DOPE and DC-chol which are used to prepare a cationic lipid microemulsion (CLM), wherein a process for formulation of a siRNA-PEG/CLM complex by electrostatic interaction between a positively charged CLM surface and a negatively charged siRNA is illustrated;
Fig. 2 shows images of CLM observed by transmission electron microscopy (TEM) with scale bar being 500nm;
Fig. 3 shows images of CLM observed by transmission electron microscopy (TEM) with scale bar being 200nm;
Fig. 4 depicts graphs illustrating cytotoxicity analysis results of a gene carrier in MDAMB435 cells in the presence of 10% serum, wherein black rectangles and white circles represent PEI 25K and CLM, respectively;
Fig. 5 illustrates characteristics of a siRNA/CLM complex, wherein measured results for sizes and Zeta potentials of a siRNA-PEG/CLM complex have a functional relation to weight ratios of DC-chol (contained in CLM)/siRNA-PEG;
Fig. 6 illustrates characteristics of a siRNA/CLM complex, wherein a gel retardation analysis result has a functional relation to weight ratios of DC-chol (contained in CLM)/siRNA-PEG and, in panel B, M and O corresponds to a marker and a control siRNA-PEG only, respectively, and the weight ratio of completed complexation of siRNA-PEG is indicated by an arrow;
Fig. 7 depicts a graph illustrating measured sizes of the siRNA-PEG/CLM complex in a RPM-I medium 1640 containing 10% serum;
Fig. 8 depicts graphs illustrating flow cytometric results of a siRNA-PEG/CLM complex labeled with cy3 in PC-3 cells after incubating for 2 hours;
Figs. 9 and 10 depict graphs illustrating that gene expression inhibition rates have functional relation to weight ratios of DC-chol (contained in CLM)/siRNA-PEG resulting from transfection of a siRNA-PEG/CLM complex.

The present invention will be more apparent from the following detailed description with accompanying drawings.

According to the first aspect of the present invention, a LDL- like cationic nanoparticle for delivering a nucleic acid comprises: a lipid core part containing cholesteryl ester and triglyceride; and a cationic surface lipid part containing cholesterol, phospholipids and cationic lipids, which forms a cationic surface of the lipid core part via hydrophobic interaction. According to the present invention, the cationic lipid is combined with the nucleic acid by electrostatic interaction to form a nucleic acid/lipid complex.

Cholesteryl ester of the present invention refers to cholesterol combined with saturated or unsaturated fatty acid having 10 to 24 carbon atoms by esterification.

Preferably, the cholesteryl ester is ester of unsaturated fatty acid having 16 to 18 carbon atoms such as oleic acid. The nanoparticle of the present invention may include single or plural kinds of cholesteryl esters.

Triglyceride of the present invention may include purified triglyceride having different compositions of various fatty acids or vegetable oils primarily containing triglyceride having plural fatty acids. Preferably, the triglyceride includes animal or vegetable oils and the vegetable oils may include soy bean oil, olive oil, cotton seed oil, sesame oil, liver oil and the like. Such oil may be used alone or in combination with two or more thereof.

The cholesteryl ester and the triglyceride of the present invention may form a lipid core part of the LDL- like cationic nanoparticle of the present invention through hydrophobic interaction.

Phospholipids of the present invention may include any kind of neutral, cationic, and anionic phospholipids and, in addition, single or plural kinds of phospholipids. The phospholipids may include phosphatidyl choline (PC), phosphatidyl ethanolamine, phosphatidyl serine, phosphatidyl glycerol, lyso types of the above phospholipids, or fully saturated or partially hardened forms having aliphatic chains with 6 to 24 carbon atoms. The phospholipids of the present invention are not particularly limited, however, may include at least one selected from a group consisting of: dioleoylphosphatidyl ethanolamine (DOPE); palmitoyloleoyl phosphatidyl choline (POPC); egg phosphatidyl choline (EPC); distearoylphosphatidyl choline (DSPC); dioleoylphosphatidyl choline (DOPC); dipalmitoylphosphatidyl choline (DPPC); dioleoylphosphatidyl glycerol (DOPG); and dipalmitoylphosphatidyl glycerol (DPPG).

Phospholipids and cholesterol of the present invention may improve gene transfection efficiency and function as a helper lipid for reducing cytotoxicity of cationic lipid composites. Phospholipids destabilize the membrane of endosome vesicles by facilitating fusion of cationic lipids of the nanoparticles with endosomal membrane phospholipids. Additionally, cholesterol provides morphological rigidity to the surface packing, thereby improving stability of the nanoparticle while the activity of the helper.

The cationic lipids of the present invention may include cationic lipids having a substantially positive charge at a specific pH such as physiological pH. According to an exemplary embodiment of the present invention, the cationic lipids may include at
least one selected from a group consisting of:3β-[N-(N',N',N'-trimethylaminoethane)carbamoyl]cholesterol (TC-cholesterol); 3β-[N-(N',N'-dimethylaminoethane)carbamoyl]cholesterol (DC-cholesterol); 3β-[N-(N'-monomethylaminoethane)carbamoyl]cholesterol (MC-cholesterol), 3β-[N-(aminoethane)carbamoyl]cholesterol (AC-cholesterol); N-(N'-aminoethane)carbamoyl propanoic tocopherol (AC-tocopherol); N-(N'-methylaminoethane)carbamoyl propanoic tocopherol (MC-tocopherol); N,N-dioleyl-N,N-dimethylammonium chloride (DODAC); N,N-distearyl-N,N-dimethylammonium bromide (DDAB); N-(1-(2,3-dioleoyloxy)propyl-N,N,N-trimethylammonium chloride (DOTAP); N,N-dimethyl-(2,3-dioleoyloxy)propylamine (DODMA); N-(1-(2,3-dioleyloxy)propyl)-N,N,N-trimethylammonium chloride (DOTMA); 1,2-dioleoyl-3-dimethylammonium-propane (DODAP) ; 1,2-dioleoylcarbamyl-3-dimethylammonium-propane (DOCDAP); 1,2-dilineoyl-3-dimethylammonium-propane (DLINDAP); dioleoyloxy-N-[2-(sperminecarboxamido)ethyl]-N,N-dimethyl-1-propanaminium-trifluoroacetate (DOSPA); dioctadecylamidoglycyl spermine (DOGS); 1,2-dimyristyloxypropyl-3-dimethyl-hydroxyethyl ammonium bromide (DMRIE), 3-dimethylamino-2-(cholest-5-en-3-beta-oxybutan-4-oxy)-1-(cis,cis-9,12-octadecadien oxy)propane (CLinDMA); 2-[5'-(cholest-5-en-3-beta-oxy)-3'-oxapentoxy]-3-dimethyl-1-(cis,cis-9',12'-octadecadienoxy)propane (CpLinDMA); N,N-dimethyl-3,4-dioleyloxybenzylamine (DMOBA); 1,2-N,N'-dioleylcarbamyl-3-dimethylaminopropane (DOcarbDAP) ; 1,2-diacyl-3-trimethylammonium-propane (TAP); and 1,2-diacyl-3-dimethylammonium-propane (DAP).

Especially, DC-chol shows lower cytotoxicity than other cationic lipids and DC-chol based gene carriers have received approval to be used in clinical therapy for various diseases including, for example, melanoma, cystic fibrosis, cervical cancer, breast cancer, ovarian cancer and so forth. Therefore, DC-chol may be preferably used in the present invention.

A preferred embodiment of the present invention is to provide a LDL-like cationic nanoparticle for delivering a nucleic acid, comprising: a lipid core part containing cholesteryl ester and triglyceride; and a cationic surface lipid part containing cholesterol, dioleoyl phosphatidyl ethanolamine (DOPE) and 3β-[N-(N',N'-dimethylaminoethane)carbamoyl]-cholesterol (DC-chol), which forms a cationic surface of the lipid core part via hydrophobic interaction.

The nanoparticle of the present invention is a LDL-like cationic nanoparticle. A natural LDL typically comprises two lipid phases, that is, polar constituents (phospholipids and apolipoproteins) and non-polar neutral lipids previously consisting of cholesterol ester and triglyceride, and composition and physicochemical characteristics thereof are shown in Table 1. Phospholipid and apolipoprotein emulsify non-polar lipid to ensure stability of a surface thereof, thereby forming a stable bio-microemulsion.

**TABLE 1. Compositions of Natural LDL and physicochemical characteristics thereof**

| Section | Natural LDL | |
|---|---|---|
| | Ingredients | Content (w/w) |
| lipid Core part | Cholesteryl ester | 45% |
| | Triglyceride | 3% |
| Surface part | Cholesterol | 10% |
| | Phospholipid | 22% |
| | Apolipoprotein B-100 | 20% |
| Size (nm) | 18 to 25 | |
| Zeta potential (mV) | -11.4±1.9[fig. 5] | |

The LDL-like cationic nanoparticle for delivering a nucleic acid according to the present invention includes: 30 to 60wt.% of cholesteryl ester; 0.1 to 10wt.% of triglyceride; 5 to 20wt.% of cholesterol; 5 to 30wt.% of phospholipids; and 10 to 50wt.% of cationic lipid. Preferably, this nanoparticle may include: 40 to 50wt.% of cholesteryl ester; 1 to 5wt.% of triglyceride; 8 to 12wt.% of cholesterol; 12 to 16wt.% of phospholipids; and 25 to 30wt.% of cationic lipid.

A weight ratio of the lipid core part to the surface lipid part in the nanoparticle of the present invention may range from 30:70 to 70:30 relative to weight of a nanoparticle carrier, preferably 40:60 to 60:40, and more preferably, 45:55 to 55:45.

In an exemplary embodiment of the present invention, as for CLM, a molar ratio of phospholipids : cholesterol : cationic lipid is 9.4 : 13 : 26 and a molar ratio of a cationic lipid to a helper lipid is 1.16, which may allow an effective composition to have a substantially equal molar ratio.

The LDL-like cationic nanoparticle of the present invention may be used for delivering a nucleic acid.

Such a nucleic acid may be selected from a group consisting of siRNA, ribosomal RNA (rRNA), RNA, deoxyribonucleic acid (DNA), complementary DNA (cDNA),aptamer, messenger DNA (mRNA), transfer RNA (tRNA) and anti-sense oligodeoxynucleotide (AS-ODN), however, is not particularly limited thereto.

For example, siRNA used herein means a duplex RNA or a single strand RNA which has a duplex RNA form inside the single strand RNA. Two strands of duplex RNAs may be combined by hydrogen bonds between nucleotides, and all nucleotides in the duplex RNA need not to be completely and complementarily combined together. A length of the siRNA may range from 15 to 60, 15 to 50, or 15 to 40 nucleotides (for a duplex RNA, the number of nucleotides at one strand, that is, the number of base pairs while, for a single strand RNA, the length of a duplex strand inside the single strand RNA). Normally, the above siRNA includes the siRNA with a length of 15 to 30, 15 to 25, or 16 to 25 nucleotides and, preferably, 19 to 25, 2 1 to 25, or 2 1 to 23 nucleotides. In addition, the siRNA may include nucleotides with different functional groups introduced therein so as to increase stability in blood or to deteriorate immune activity.

Accordingly, the siRNA of the present invention may be a modified or un-modified form of a typical siRNA. For example, one terminal of the siRNA may be modified with polyethylene glycol (PEG).

PEG is a hydrophilic, flexible and non-ionic polymer and is one of generally known substances that modify the surface of nanoparticles and allow a carrier to have a long circulation cycle in order to prevent a mononuclear phagocyte system (MPS) from recognizing the surface of nanoparticles (Xing X, Yujiao Chang J, Hung M.

Preclinical and clinical study of HER-2/neu-targeting cancer gene therapy, Adv Drug Deliv Rev. 30(1-3) (1998) 219-227.; S. Mao, M. Neu, O. Germershaus, O. Merkel, J. Sitterberg, U. Bakowsky, T. Kissel., Influence of polyethyleneglycol chain length on the physicochemical and biological properties of poly(ethyleneimine)-graft-poly(ethyleneglycol) block copolymer/SiRNA polyplexes, Bioconjug Chem. 17 (5) (2006) 1209-18).

In an exemplary embodiment of the present invention, if PEG has a molecular weight of 5,000 dalton, the siRNA can be sufficiently protected against RNase digestion and, at the same time, continuously maintain superior transfection performance of the same.

In the present invention, N/P ratio of the cationic lipid to the nucleic acid may range 0.1 to 128, preferably 0.5 to 32, and more preferably, 1 to 16. In an exemplary embodiment of the present invention, a weight ratio of the cationic lipid to the nucleic acid may range from 1.4 to 32, and preferably, 2.8 to 16.8.

The LDL-like cationic nanoparticle of the present invention may include one or plural kinds of apoproteins. The apoprotein may be extracted from a natural lipoprotein or produced by recombination of proteins. Preferred examples of the apoprotein may include B-100, apo E, etc. Such an apoprotein may allow the nanoparticle of the present invention to be efficiently introduced into cells in a specific mode.

According to a second aspect of the present invention, there is provided a method for preparing a LDL-like cationic nanoparticle for delivering a nucleic acid. Kinds and contents of constituents in the nanoparticle prepared by this method are substantially identical to those described above.

In an exemplary embodiment of the present invention, there is provided a method for preparing a LDL-like cationic nanoparticle for delivering a nucleic acid comprising: (a) dissolving cholesteryl ester, triglyceride, phospholipids, cholesterol and a cationic lipid in an organic solvent; (b) removing the organic solvent to generate a lipid film;(c) adding a water soluble solution to the lipid film to hydrate the same; and (d) complexing the resulting LDL-like cationic nanoparticle with the nucleic acid.

The organic solvent used in the step (a) may include, for example, at least one selected from a group consisting of chloroform, methanol and cyclohexane. For example, the organic solvent is chloroform or methanol alone or a combination thereof in a relative ratio, however, is not particularly limited thereto.

The organic solvent in the step (b) is removed at a temperature of higher than a melting point of cholesteryl ester and, if cholesteryl oleat is used, the temperature may preferably range from 52 to 60ºC.

In another exemplary embodiment of the present invention, there is provided a method for preparing a LDL-like cationic nanoparticle for delivering a nucleic acid comprising: (a') dissolving cholesteryl ester, triglyceride, phospholipids, cholesterol and a cationic lipid; (b') adding water to the above solution and mixing the same to prepare a mixture; (c') agitating the mixture to form a homogeneous solution; (d') complexing the resulting LDL-like cationic nanoparticle with the nucleic acid.

In the step (a'), the lipid ingredient may be dissolved by heating or using the organic solvent in the step (a) described above. In case of dissolving the lipid ingredient by using the organic solvent, the above method according to the second exemplary embodiment may further comprise a step of removing the organic solvent from the homogeneous solution. The organic solvent is removed at a temperature of higher than a melting point of cholesteryl oleate and, if cholesteryl ester is used, the temperature may preferably range from 52 to 60ºC.

In the step (b'), water may be added in an amount of 3 to 7 times (v/v) the solution. The agitation in the step (c') may be performed by any conventional method such as sonication, high pressure homogenization, use of a membrane fluidizer, etc. to produce uniform particles. As for sonication, it may be carried out at 125W for 1 to 5 minutes, however, these conditions are not particularly limited thereto.

Preferably, the LDL-like cationic nanoparticle for delivering a nucleic acid according to another exemplary embodiment of the present invention may be prepared by modified solvent-emulsification.

In a third aspect of the present invention, there is provided a method for delivering a nucleic acid to a target cell using the LDL-like cationic nanoparticle for delivering a nucleic acid described above.

In an exemplary embodiment of the present invention, there is provided a method comprising: (1) preparing a complex of a nucleic acid and the LDL-like cationic nanoparticle as described above; and (2) transfecting the prepared complex to a target cell.

The complex in the step (1) may be formed by incubating the LDL-like cationic nanoparticle in phosphate buffered saline (PBS) or desalted water in the presence of nucleic acid. The PBS may have pH 7.0 to 8.0 and include 0.8% NaCl, however, is not particularly restricted thereto.

The above method may further comprise a gel retardation process of the complex resulting from a nucleic acid-PEG conjugate and the LDL-like cationic nanoparticle after the step (1). Such a gel retardation process may be performed in order to determine whether the complex is stably formed by electrostatic interaction between a negatively charged siRNA and a positively charged surface of nanoparticle.

In another exemplary embodiment of the present invention, if the nucleic acid is modified using PEG, the above method may further comprise a step (V) of forming a nucleic acid-PEG conjugate before the step (1).

Particularly, the conjugate in the step *(V)* may be obtained using a disulfide bond between a nucleic acid and a PEG, and more preferably, is prepared using a nucleic acid with functionalized hexylamine group at 3' end of a sense strand of siRNA. An excess N-succinimidyl-3-(2-pyridylodithio)propionate (SPDP) reacts with hexylamine at 3' end of siRNA in PBS (pH 7.5) to activate the nucleic acid. The remaining unreacted SPDP is removed using a desalting column. The SPDP-activated siRNA may excessively react with a PEG (with a molecular weight of 5,00O)-SH to generate a disulfide bond, thus being conjugated to the PEG. The remaining unreacted PEG-SH is removed by dialysis (MWCO=10,000), thereby obtaining a purified siRNA which is conjugated to the PEG by the disulfide bond.

For example, the siRNA, owing to its favorable expression inhibition activity, is very useful for gene therapy. However, this nucleic acid has problems in stability and transfection efficiency, thus being restricted in practical applications.

A cationic nanoparticle (CLM) of the present invention may form a stable complex together with a nucleic acid by electrostatic interaction in a medium containing serum, wherein the CLM combined with the nucleic acid exhibits very low cytotoxicity and excellent cellular uptake, thereby it is very useful for delivering the nucleic acid.

Alternatively, a plasma apolipoprotein may be adsorbed to a surface of CLM, thus making the CLM to imitate lipoprotein containing a natural apolipoprotein. Accordingly, it is expected that the CLM is generated in vivo by a natural lipoprotein mechanism.

### EXAMPLES

Hereinafter, the present invention will become apparent from following examples and experimental examples, which are only given for the purpose of illustration and are not to be construed as limiting the scope of the invention.

Cholesteryl oleate, glyceryl trioleate (triglyceride) and unesterified cholesterol used in the examples and/or experimental examples were commercially available from Sigma Chemical.

L-alpha-dioleoyl phosphatidyl ethanolamine (DOPE) and 3β-[N-(N',N'-dimethylaminoethane)carbamoyl]-cholesterol hydrochloride were manufactured by Avanti Polar Lipids.

In addition, VEGF siRNA and GFP siRNA were purchased from Bioneer Co.(Daejeon, Korea). A sense strand of GFP siRNA is 5'-GCAAGCUGACCCUGAAGUUdTdT-3' while an anti-sense strand thereof is 5'-AACUUCAGGGUCAGCUUGCdTdT-S'. Likewise, a sense strand of VEGF siRNA is 5'-GGAGUACCCUGAUGAGAUCdTdT-S' while an anti-sense strand thereof is 5'-GAUCUCAUCAGGGUACUCCdTdT-S'.

N-succinimidyl-3-(2-pyridyldithio)propionate (SPDP) and sulfhydryl group derived methoxy-poly(ethyleneglycol) (mPEG-SH, a molecular weight of 5,000) were purchased from Pierce (Lockford, IL) and Nectar (Huntsville, AL), respectively.

A fetal bovine serum (FBS), a Roswell Park Memorial Institute-1 (RPM-1) medium 1610, and a Dulbecco's modified Eagle's medium (DMEM) were commercially available from Gibco BRL (Grand Island, NY). In addition, other chemicals and reagents for analysis were used in the exemplary embodiments of the present invention.

### EXAMPLE 1 : Preparation of cationic lipid microemulsion

A cationic lipid microemulsion (CLM) was prepared by modified- solvent emulsification. More particularly, 22.5mg (45wt.%) of cholesteryl oleate, 1.5mg (3wt.%) of glyceryl trioleate, 7mg (14wt.%) of DOPE, 5mg (10wt.%) of unesterified cholesterol and 14mg (28wt.%) of DC-cholesterol were dissolved in 2mL of a solvent consisting of chloroform : methanol (2: 1) in a glass bottle. In CLM, a molar ratio of DOPE : cholesterol : DC-chol is 9.4 : 13 : 26 and a molar ratio of a cationic lipid to a helper lipid is 1.16, which may allow an effective composition to have a substantially equal molar ratio.

10mL of distilled water was added to the solution with sufficient stirring. The obtained suspension was subjected to sonication for 3 minutes by means of Branson 450 sonicator (20kHz, duty cycle = 40, output control = 3.5). After the prepared microemulsion was moved to a rotational evaporator, the solvent was removed at a temperature of 52 to 60ºC, which is a melting point of cholesteryl oleate, and was stored at 4ºC.

### EXAMPLE 2 : Synthesis of siRNA-PEG

A siRNA-PEG was conjugated via disulfide bonds. That is, 300µg of siRNA which had been modified by a hexylamine group at 3'-terminal of a sense strand (20nmol of VEGF or GFP siRNA), was dissolved in a PBS (pH 7.5).

Following this, 20µL (400nmol) of 20mM SPDP solution in DMSO was added to the prepared siRNA solution. After reacting the mixture at room temperature for 3 hours, excess SPDP was removed through gel permeation chromatography (D-Salt TM dextran desalting column, Pierce, Lockford, IL) to obtain purified siRNA-SPDP.

Next, 4µmol of mPEG-SH in PBS (pH 7.5) was added to the purified siRNA-SPDP,followed by reacting at room temperature for 3 days. The unreacted PEG was separated by dialysis against desalted water (MWCO 10,000), while a siRNA-PEG conjugate was concentrated using a high-speed vacuum concentration device.

Purity and concentration of the resulting product were determined by measuring UV absorption at 260 and 280nm. The purified siRNA-PEG conjugate was stored at - 80ºC.

### EXAMPLE 3: Formation of complex

Using a siRNA-PEG in each DC-chol (contained in CLM)/siRNA in 0, 1.4, 2.8, 4.2, 5.6 and 8.4 weight ratios, respectively, CLM was incubated in a PBS (pH 7.4 and 150mM NaCl) or desalted water at room temperature for 15 minutes. After that, the resulting complex was subjected to gel retardation and determination of characteristics by measuring size and Zeta potential thereof.

### EXAMPLE 4: Gel retardation of siRNA-PEG/cationic microemulsion complex

Each siRNA/CLM complex (20µL) was prepared with different weight ratios as described above and mixed with 2µL of a loading dye (10x). Loading 22µL of the whole suspension with a tris-acetate (TAE) running buffer on a well with 2% agarose gel for electrophoresis, the well was moved from a cathode to an anode at 100V for 15 minutes.

The obtained siRNA-PEG was dyed using ethidium bromide so as to be visible and a gel image thereof was obtained through UV rays.

### EXAMPLE 5: Transfection of siRNA-PEG/CLM complex

Cell culture was performed with PC3 cells (human prostate cancer cell line) obtained from Korean Cell Line Bank (Seoul, Korea) and cells were grown in a RPM-1 medium 1610 containing a heat-inactivated 10% (v/v) fatal bovine serum, 100 UI/mL of penicillin and 100µg/mL of streptomycin.

Overexpressed GFP and stably transfected MDAMB 435 cells (human breast cancer cells) provided from Samyang Co. (Daejeon, Korea) were grown in DMEM supplemented with 10% serum and the antibiotics described above.

The cells were maintained at 37ºC under a 5% CO2 in a humidified atmosphere, followed by normal division of cell culture using trypsin/EDPA.

The cy3-labeled siRNA-PEG was complexed with CLM, and then incubated for 2 hours with PC3 cells in a medium containing 10% serum, thereby attaining transfection thereof.

### EXPERIMENTAL EXAMPLE 1: Characterization of cationic nanoparticle

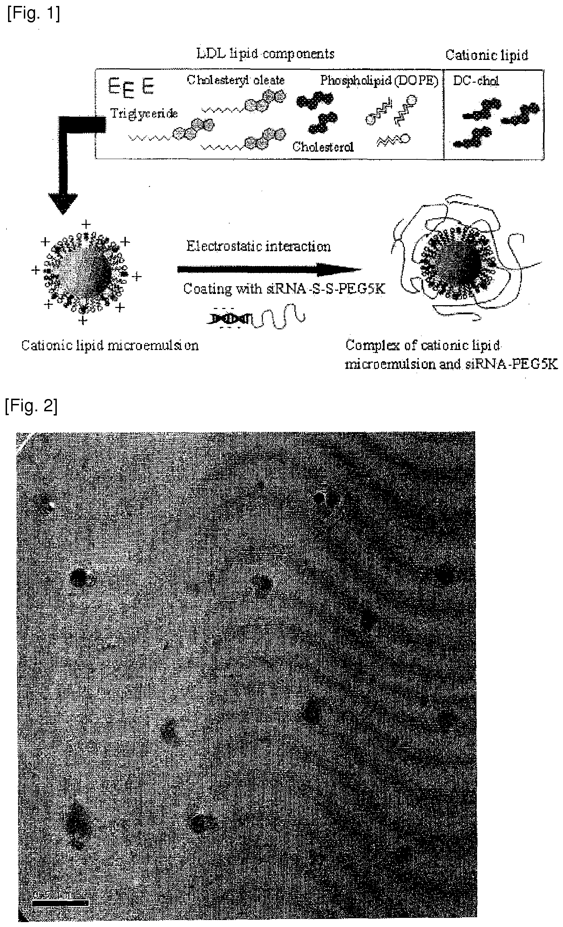

An average diameter of the CLM prepared in EXAMPLE 3 was 103.6±4.5nm as measured by laser scattering method and the CLM was observed by Transmission Electron Microscopy (TEM) to have a spherical morphology as shown Fig. 2. The CLM has Zeta potential ranging from 41.76±2.63mV. Compared to the natural LDL, this value was increased by combination of the DC-chol with the DOPE and surface charge of the CLM was altered from a negative value to a positive one. After preparation, the CLM was stably maintained at room temperature over several weeks without aggregation.

Cholesteryl oleate as a primary core composition has a melting point of 52ºC and the CLM core is present in solid state at regular physiologic temperatures. This is the reason why the stability of the CLM is maintained over the long term. The CLM having high stability may be more advantageous than conventional DC-chol/DOPE liposome formulations.

As for physicochemical characteristics of the CLM, it was identified that the CLM is a lipid microemulsion positively modified for delivering siRNAs and may be a stable LDL-like system useful for medical treatment applications.

The morphology of the CLM was visibly observed by TEM. 20µL of the microemulsion (5mg/mL) was immersed into a Formvar/carbon support grid with a size of 300 mesh three times in sequence, followed by drying the grid for 2 minutes. The resulting grid was observed by Zeiss Omega 912 TEM (Car Zeiss, Oberkochen, Germany) at 80kV.

Measurement of the size and the zeta potential was performed using a dynamic light scatter (DLS) equipped with a He-Ne laser at wavelength of 632nm and a detection angle of 90º (Zeta-Plus, Brookhaven Instruments, NY) to determine a diameter and a surface zeta potential of the CLM and/or a complex of the CLM with the siRNA-PEG.

As to size measurement, each sample was desirably diluted in a desalted water to maintain the number of counts per second between 104 and 105. In order to study stability of a complex in serum, a RPM-1 medium 1640 containing 10% FBS was added to the complex and was subjected to measurement in terms of reaction kinetics.

### EXPERIMENTAL EXAMPLE 2: Cytotoxicity analysis of cationic nanoparticle

As for determination of cytotoxicity of the cationic nanoparticle, MDAMB 435 cells were seeded on a 96-well plate (104 cells per well) 24 hours before cytotoxicity analysis.

Each of CLMs and PEIs with different concentrations (3, 6, 12, 18, 24, 36, 48 and 72µg/mL) was prepared in a RPM-1 medium 1610 containing 10% FBS. After removing the medium, 100µL of the prepared suspension was added to each well of the plate. Incubating the cells with the CLM or PEI suspension at 37ºC for 24 hours, 10µL of a Cell Counting Kit-8 (CCK-8) solution (Dojindo molecular technologies Inc., MD, USA) was added to each well. Incubating the cells at 37ºC for an additional 4 hours, the culture medium was subjected to measurement of absorption at 450nm using a micro-plate (BioRad Model 550).

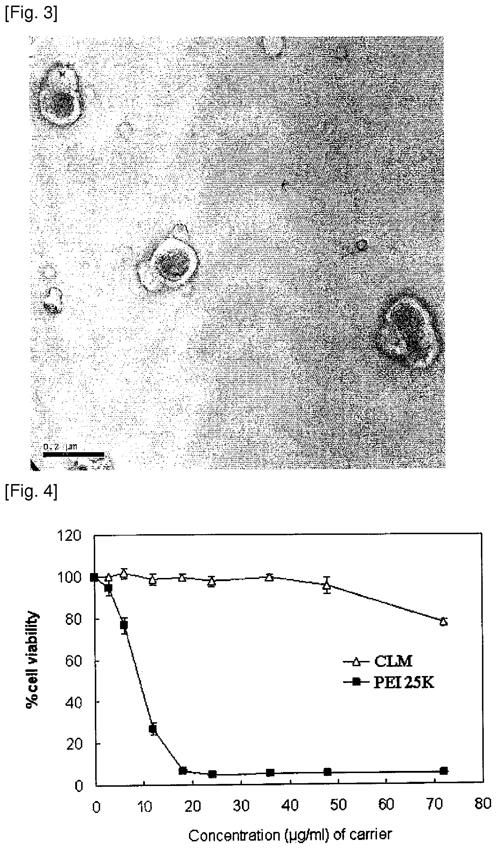

In vitro cytotoxicity analysis was performed by releasing a cellular dehydrogenase of MDAMB 435 cells to the culture medium and quantifying the results. In the presence of medium containing 10% serum, the CLM or the PEI 25K as one of the most popular non- viral type of gene carriers was used in an amount of 3 to 72µg/mL for the above analysis, in consideration of following transfection experiments. PEI 25K demonstrated only 6.9±0.4% cell viability at 18µg/mL after 24 hours incubation (IC50 value was about 9µg/mL). While the CLM substantially exhibited no harmful influence at up to 48µg/mL. As for high dose administration, 72µg/mL of CLM exhibited cell viability of 78±1%, which is considerably higher than the PEI 25K showing cell viability of 5.2±0.1% (see Fig. 3).

As a result of comparing cytotoxicity between siRNA-free PEI 25K and CLM, the CLM exhibited lower cytotoxicity than the PEI 25K. This suggested that the CLM may be more advantageous than the PEI in terms of cytotoxicity, may have stable transfection efficiency and preferably replace the PEL

### EXPERIMENTAL EXAMPLE 3: Characterization of siRNA-PEG/CLM complex

As for characterization of a complexation between a siRNA-PEG and a CLM, the siRNA-PEG was incubated with CLM in an aqueous solution at room temperature in a functional relationship to weight ratio of DC-chol (contained in CLM)/siRNA. Complexing degree through electrostatic interaction was determined by measuring a size of a microemulsion and a zeta potential of the same according to DLS.

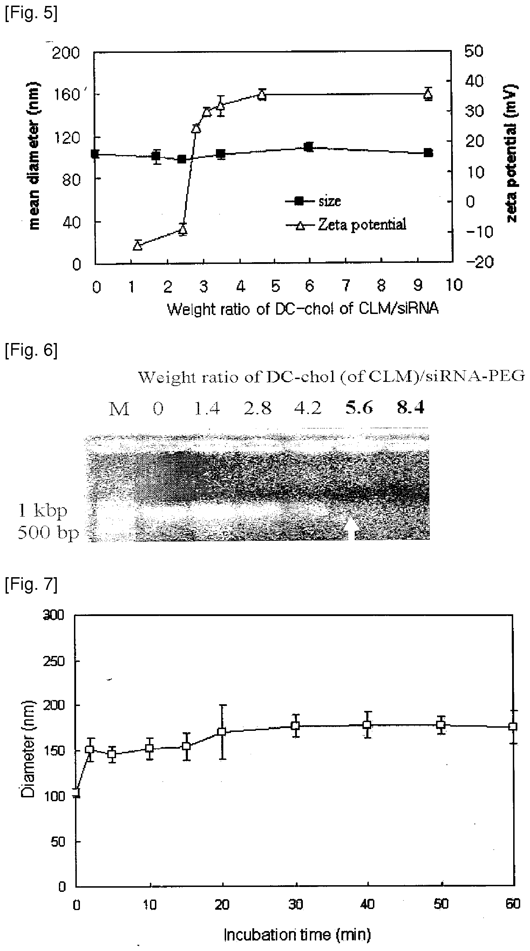

As illustrated in Fig. 5, as the weight ratio of DC-chol (contained in CLM)/siRNA increased from 1 to 4.67, the zeta potential of the CLM increased from -13.8 ±3.9mV to +35.67±1.2mV, and then, this value was maintained when the weight ratio of DCchol/siRNA exceeded 4.7. Such data suggested that all negatively charged siRNA phosphate residues form completely complex with CLM at the DC-chol/siRNA weight ratio of 4.7.

Furthermore, it was observed that the size of the CLM coated with the siRNA-PEG was substantially maintained near 100nm regardless of the weight ratio of DC-chol(contained in CLM)/siRNA-PEG, and therefore, the CLM had not aggregated so much after incubating with the siRNA-PEG (see Fig. 5). From these size and zeta potential data, it was demonstrated that a positively charged surface of the siRNA-PEG/CLM complex may generate charge repulsion between complexes.

In addition, it was determined that PEG chains on the surface of the complex served as a protective cloud and prevented aggregation of the CLM by steric repulsion.

Simultaneously with this, a gel retardation analysis was performed for the DC-chol (contained in CLM)/siRNA at the weight ratio in a range of 1.4 to 8.4. As a result of agarose gel electrophoresis, it was found that the CLM was sufficiently retarded by the siRNA-PEG at the weight ratio of 5.6 and 8.4 (see Fig. 6).

These results demonstrated that when the weight ratio exceeds 5.6, the siRNAPEG/CLM complex is sufficiently formed through interaction of charges. The zeta potential data and the gel retardation analysis proved that the complexation was completed at the weight ratio of about 5, thus supporting inter-relation between the complexation and the weight ratio of the siRNA-PEG/CLM complex.

### EXPERIMENTAL EXAMPLE 4: Stability of siRNA-PEG/CLM complex in medium containing 10% serum

The PEG 5K was introduced to a siRNA delivery system by conjugation through disulfide bonds. After a siRNA-PEG/CLM complex was produced, a size of this complex was measured in medium containing 10% serum using DLS.

As shown in Fig. 7, two kinds of adsorption modes, that is, fast and slow adsorption of plasma proteins were represented in terms of kinetics. Fast adsorption was attained later than 2 minutes of incubation time during which the size of the complex was increased to 47.6nm. When the incubation was performed in medium containing serum for 2 to 20 minutes, the size of the siRNA-PEG/CLM complex slowly but gradually grew to 19.3nm (for example, 151.2±13.2nm at 2 minutes and 170.3±29.9nm at 20 minutes), showing a specific alteration profile thereof.

It was demonstrated that absorption and re- arrangement of particles on a surface of polyethylene glycolated (PEGylated) nanoparticle system of a plasma protein were initially increased and, after incubating for 20 minutes, reached maximum levels thereof. Such facts explained adsorption of the protein on the siRNA-PEG/CLM complex during the first 20 minute incubation. Further, when the incubation proceeded for 20 to 60 minutes, the size of the complex was substantially unchanged without an increase in size caused by aggregation, thus exhibiting stability of the complex.

The results obtained above suggested that, steric repulsion due to PEG chains on LDL-mimicking nanoparticles may reduce discrete adsorption of a plasma protein and may contribute high stability in medium containing serum without aggregation phenomenon.

### EXPERIMENTAL EXAMPLE 5: Cellular uptake of siRNA/CLM complex

For evaluation of cellular uptake capability of a siRNA/CLM complex, a cy3-labeled siRNA-PEG was complexed with CLM, and then incubated for 2 hours with PC3 cells in 10% serum containing medium. The resultant cy3- siRNA-PEG/CLM complex was subjected to analysis of relative cellular uptake flow cytometry.

More particularly, PC3 cells were seeded at a density of 5x105 cells per well on six(6) cell plates, which are in a RPM-1 medium 1640 containing 10% FBS and an antibiotic, and grown at 37ºC for 24 hours. The obtained cells were washed with a PBS (pH7.4) after removing the medium lug of siRNA-PEG or siRNA-PEG/CLM complex (with 8.4 weight ratio of DC-chol (contained in CLM)/siRNA) was incubated at 37ºC for 2 hours. Removing the medium, the cellular uptake was stopped. The obtained cells were gently washed with a cold PBS, followed by fixing the cells using 1% (w/v) paraformaldehyde solution. The cellular uptake was observed by flow cytometry (FACScan, Becton, Dickinson) and analyzed using CELLQUEST software (PharMingen).

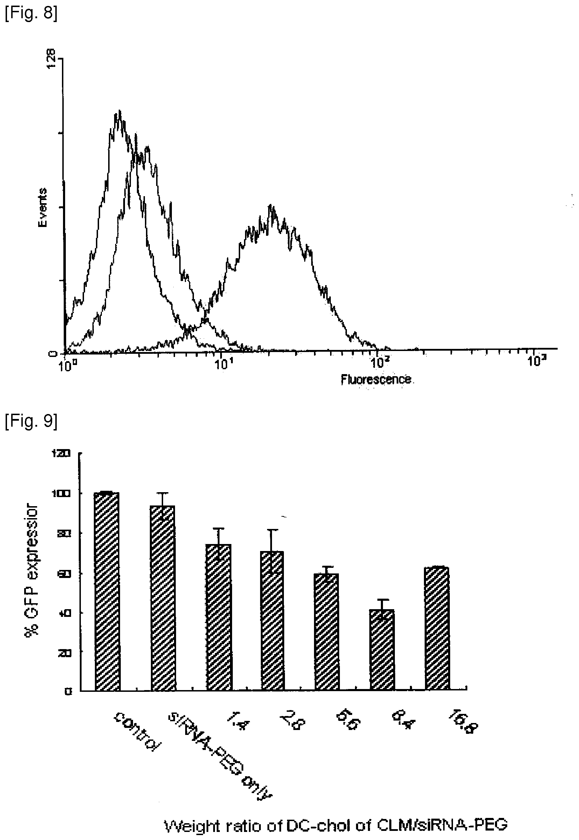

Fig. 8 shows that a fluorescence intensity profile of the cy3-siRNA-PEG/CLM complex was shifted to the right in the graph. This result demonstrated that the cellular uptake was considerably greater than that of a control.

In the case of cy3-siRNA-PEG only, there is a slight shift in the cellular uptake result, compared to the control. Therefore, it was presumed that the cy3-siRNA-PEG may be self-formed and micelles may cause a small increase of the cellular uptake.

### EXPERIMENTAL EXAMPLE 6: Transfection efficiency of siRNA-PEG/CLM complex

Efficiency of inhibiting siRNA gene of the siRNA-PEG/CLM complex was determined using a stable MDAMB 435 cell line, which does over-expresion of GFP, and was transfected in serum containing medium.

More particularly, GFP-overexpressing MDAMB 435 cells, were seeded on twelve (12) cell plates at a density of 2x105 cells per well, which contained 10% FBS and an antibiotic in a DMEM medium, and cultured 24 hours before a transfection experiment. The obtained cells were washed with a PBS (pH7.4) three times after removing the medium.

1µg of each siRNA-PEG or siRNA-PEG/CLM complex with different weight ratio of DC-chol (contained in CLM)/siRNA (ratio of 0, 1.4, 2.8, 5.6, 8.4 and 16.8) was transfected in medium containing 10% FBS at 37ºC for 2 hours. The cell supernatant was replaced by new medium supplemented with serum.

Next, the transfected cells were grown for 42 hours and treated using 0.1 % Triton X100 in a PBS. A cell lysate was subjected to fluorescence measurement at 525nm (the lysate exhibited excitation at 488nm).

As for a VEGF release inhibition experiment using PC3 cells, a sample was transfected as described above and, after 4 hours, the used cell medium was replaced by new medium containing serum. After culturing for 6 hours following the transfection treatment, the used medium was again replaced by new RPM- 1 medium supplemented with 10% FBS and 20µg/mL of heparin. After incubating the sample for an additional 16 hours, the cell supernatant containing VEGF was collected.

A concenttration of VEGF released from the cells was determined using a Quantikine human VEGF-immunoassay kit (R&D system, Minneapolis, MN) according to instructions of a manufacturer.

The GFP or VEGF based siRNA-PEG/CLM complex was treated using GFPoverexpressing MDAMB435 cells or VEGF releasing PC-3 cells, respectively, in RPM-1 medium containing 10% serum.

Fig. 9 showed efficiency of silencing GFP gene of the above complex. The siRNAPEG/CLM complex inhibited expression of GFP gene at the weight ratio ranging from 1.4 to 8.4 according to increase in weight ratio of DC-chol (contained in CLM)/siRNA.

At each weight ratio of 5.6 and 8.4, the CLM complex containing the GFP based siRNA-PEG showed a down regulation rate of 41.2±3.7% and 58.9±4.9%, respectively, in terms of expression of GFP. This result substantially corresponds to a fact that a GFP based siRNA-PEG/CLM complex was completely formed in a case that the weight ratio exceeds 5.6 (see Fig. 9).

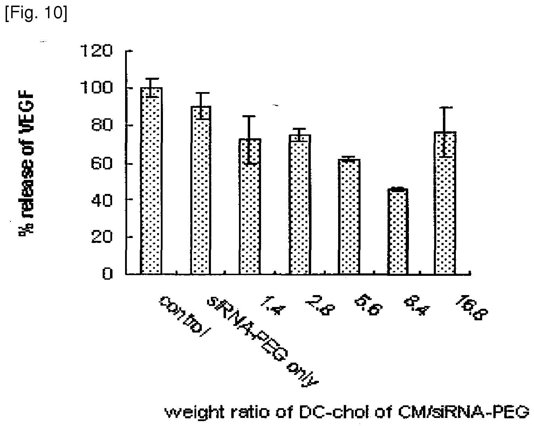

It is possible to explain that performance of silencing gene can be improved by the complete complex as a delivery system having stoichiometric effects to siRNA. Transfection efficiency was also determined in VEGF releasing PC3 cells by the same method as described above except that a VEGF based siRNA-PEG was used in a complexation with the CLM. VEGF inhibition profiles in PC3 cells were substantially identical to GFP inhibition profiles in MDAMB435 cells (see Fig. 10).

As for VEGF based siRNA-PEG/CLM complexes at the weight ratios of 5.6 and 8.4, VEGF expression inhibition rates were 37.6±1.2% and 53.8±0.9%, respectively. These results were presented in PC3 cells in sequence. Meanwhile, efficiency of silencing gene of siRNA-PEG only were 6.9±6.5% and 9.55±7.2%, respectively, since each of GFP based siRNA-PEG and VEGF based siRNA-PEG was shown the low cellular uptake capability (see Figs 9 and 10.)

Inhibition rates of target protein expression at a weight ratio of 16.8 for GFP based siRNA-PEG/CLM and VEGF based siRNA-PEG/CLM complexes were 38±0.3% and 22.8±13%, respectively. These results suggested that un-complexed CLMs may remain and compete with the siRNA-PEG/CLM complex in view of cellular uptake. This may be the reason why the efficiency of silencing gene is slightly reduced at the weight ratio of 16.8.

As is apparent from the description disclosed above, LDL receptors are overexpressed in various cancers including, for example, myelogenous leukemia cells, intestinal cancer, renal cancer, brain cancer and so forth, and therefore, a cationic nanoparticle of the present invention is effectively used in cancer therapy using such LDL receptors for treatment of the above diseases.

## Claims

1. Complex of a nucleic acid and a low density lipoprotein (LDL)-like cationic nanoparticle for delivering a nucleic acid comprising: a lipid core part containing cholesteryl ester and triglyceride; and a cationic surface lipid part containing cholesterol, phospholipids and a cationic lipid, which forms a cationic surface of the lipid core part via hydrophobic interaction, wherein the cationic lipid is combined with the nucleic acid via electrostatic interaction.

2. Complex according to claim 1, wherein the cationic nanoparticle includes 30 to 60 wt.% of cholesteryl ester, 0.1 to 10 wt.% of triglyceride, 5 to 20 wt.% of cholesterol, 5 to 30 wt.% of phospholipids and 10 to 50 wt.% of cationic lipid.

3. Complex according to claim 1 or 2, wherein a weight ratio of the lipid core part to the cationic surface lipid part in the nanoparticle ranges from 30:70 to 70:30.

4. Complex according to any of the preceding claims, wherein the phospholipids are at least one selected from a group consisting of:
dioleoylphosphatidyl ethanolamine (DOPE); palmitoyloleoyl phosphatidyl choline (POPC); egg phosphatidyl choline (EPC); distearoylphosphatidyl choline (DSPC);
dioleoylphosphatidyl choline (DOPC); dipalmitoylphosphatidyl choline (DPPC);
dioleoylphosphatidyl glycerol (DOPG); and dipalmitoylphosphatidyl glycerol (DPPG).

5. Complex according to any of the preceding claims, wherein the cationic lipid is at least one selected from a group consisting of:
3β-[N-(N',N',N'-trimethylaminoethane)carbamoyl]cholesterol (TC-cholesterol); 3β-[N-(N',N'-dimethylaminoethane)zarbamoyl]cholesterol (DC-cholesterol); 3β-[N-(N'-monomethylaminoethane)carbamoyl]cholesterol (MC-cholesterol), 3β-[N-(aminoethane)carbamoyl]cholesterol (AC-cholesterol); N-(N'-aminoethane)carbamoyl propanoic tocopherol (AC-tocopherol); N-(N-methylaminoethane)carbamoyl propanoic tocopherol (MC-tocopherol); N,N-dioleyl-N,N-dimethylammonium chloride (DODAC); N,N-distearyl-N,N-dimethylammonium bromide (DDAB); N-(1-(2,3-dioleoyloxy)propyl-N,N,N-trimethylammonium chloride (DOTAP); N,N-dimethyl-(2,3-dioleoyloxy)propylamine (DODMA); N-(1-(2,3-dioleyloxy)propyl)-N,N,N-trimethylammonium chloride (DOTMA); 1,2-dioleoyl-3-dimethylammonium-propane (DODAP); 1,2-dioleoyl carbamyl-3-dimethylammonium-propane (DOCDAP) ; 1,2-dilineoyl-3-dimethylammonium-propane (DLINDAP); dioleoyloxy-N-[2-(sperminecarboxamido)ethyl] -N,N-dimethyl-1-propanaminium-trifluoroacetate (DOSPA); dioctadecylamidoglycyl spermine (DOGS); 1,2-dimyristyloxypropyl-3-dimethyl-hydroxyethyl ammonium bromide (DMRIE), 3-dimethylamino-2-(cholest-5-en-3-beta-oxybutan-4-oxy)-1-(cis,cis-9,12-octade cadienoxy)propane (CLinDMA); 2-[5'-(cholest-5-en-3-beta-oxy)-3'-oxapentoxy]-3-dimethyl-1-(cis,cis-9',12'-octadecadienoxy)propane (CpLinDMA); N,N-dimethyl-3,4-dioleyloxybenzylamine (DMOBA) ; 1,2-N,N'-dioleylcarbamyl-3-dimethylaminopropane (DOcarbDAP) ; 1,2-diacyl-3-trimethylammonium-propane (TAP); and 1,2-diacyl-3-dimethylammonium-propane (DAP).

6. Complex according to any of the preceding claims, wherein the nucleic acid is selected from a group consisting of small interfering RNA (siRNA), ribosomal RNA (rRNA), ribonucleic acid (RNA), deoxyribonucleic acid (DNA), complementary DNA (cDNA), aptamer, messenger DNA (mRNA), transfer RNA (tRNA) and antisense oligodeoxynucleotide (AS-ODN).

7. Method for preparing a LDL-like cationic nanoparticle for delivering a nucleic acid comprising:
a) dissolving cholesteryl ester, triglyceride, phospholipids, cholesterol and a cationic lipid in an organic solvent;
b) removing the organic solvent from the solution to form a lipid film;
c) adding a water soluble solution to the lipid film to hydrate the same; and
d) complexing the resulting LDL-like cationic nanoparticle with the nucleic acid.

8. Method for preparing a LDL-like cationic nanoparticle for delivering a nucleic acid comprising:
a') dissolving cholesteryl ester, triglyceride, phospholipids, cholesterol and a cationic lipid;
b') adding water to the above solution and mixing the same to prepare a mixture;
c') agitating the mixture to form a homogeneous solution; and
d') complexing the resulting LDL-like cationic nanoparticle with the nucleic acid.

9. Method according to claim 8, wherein the agitation in the step (c') is carried out by any process selected from sonication, high pressure homogenization and use of a membrane fluidizer.

10. Method according to claim 8 or 9, further comprising a step of removing the organic solvent after the step (c').

11. Method according to any of claims 8-10, wherein cholesteryl ester, triglyceride, phospholipids, cholesterol and the cationic lipid are dissolved by heating.

12. Method according to any of claims 8-11, wherein cholesteryl ester, triglyceride, phospholipids, cholesterol and the cationic lipid are dissolved by adding an organic solvent thereto.

13. Method according to any of claims 7-12, wherein the organic solvent is at least one selected from a group consisting of chloroform, methanol and cyclohexane.

14. Method according to any of claim 7-13, wherein the complexing step (d, d') comprising incubating the LDL-like cationic nanoparticle in phosphate buffered saline or desalted water in the presence of the nucleic acid.

## Patentansprüche

1. Komplex einer Nukleinsäure und eines Lipoproteinniederer-Dichte(LDL)-artigen kationischen Nanopartikels zum Abgeben einer Nukleinsäure, umfassend: ein Lipid-Kernpartikel, das Cholesterylester und Triglycerid enthält; und einen kationischen Oberflächenlipidteil, der Cholesterol, Phospholipide und ein kationisches Lipid enthält und der über hydrophobe Wechselwirkung eine kationische Oberfläche des Lipid-Kernteils bildet, wobei das kationische Lipid über elektrostatische Wechselwirkung mit der Nukleinsäure kombiniert ist.

2. Komplex gemäß Anspruch 1, wobei das kationische Nanopartikel 30 bis 60 Gew.-% Cholesterylester, 0,1 bis 10 Gew.-% Triglycerid, 5 bis 20 Gew.-% Cholesterol, 5 bis 30 Gew.-% Phospholipide und 10 bis 50 Gew.-% kationisches Lipid enthält.

3. Komplex gemäß Anspruch 1 oder 2, wobei das Gewichtsverhältnis des Lipid-Kernteils zu dem kationischen Oberflächenlipidteil in dem Nanopartikel in dem Bereich von 30:70 bis 70:30 liegt.

4. Komplex gemäß einem der vorstehenden Ansprüche, wobei die Phospholipide wenigstens eines ausgewählt aus der Gruppe bestehend aus: Dioleoylphosphatidylethanolamin (DOPE); Palmitoyloleoylphosphatidylcholin (POPC); Phosphatidylcholin aus Ei (EPC); Distearoylphosphatidylcholin (DSPC); Dioleoylphosphatidylcholin (DOPC); Dipalmitoylphosphatidylcholin (DPPC); Dioleoylphosphatidylglycerol (DOPG); und Dipalmitoylphosphatidylglycerol (DPPG) sind.

5. Komplex gemäß einem der vorstehenden Ansprüche, wobei das kationische Lipid wenigstens eines ausgewählt aus der Gruppe bestehend aus: 3β-[N-(N',N',N'-Trimethylaminoethan)carbamoyl]-cholesterol (TC-Cholesterol); 3β-[N-(N',N'-Dimethylaminoethan)carbamoyl]cholesterol (DC-Cholesterol); 3β-[N-(N'-Monomethylaminoethan)-carbamoyl]cholesterol (MC-Cholesterol), 3β-[N-(Aminoethan)carbamoyl]cholesterol (AC-Cholesterol); N-(N'-Aminoethan)carbamoylpropansäuretocopherol (AC-Tocopherol); N-(N-Methylaminoethan)carbamoylpropansäuretocopherol (MC-Tocopherol); N,N-Dioleyl-N,N-dimethyl-ammoniumchlorid (DODAC); N,N-Distearyl-N,N-dimethylammoniumbromid (DDAB); N-(1-(2,3-Dioleoyloxy)propyl-N,N,N-trimethylammoniumchlorid (DOTAP); N,N-Dimethyl-(2,3-dioleoyloxy)propylamin (DODMA); N-(1-(2,3-Dioleyloxy)propyl)-N,N,N-trimethylammoniumchlorid (DOTMA); 1,2-Dioleoyl-3-dimethylammoniumpropan (DODAP); 1,2-Dioleoylcarbamyl-3-dimethylammoniumpropan (DOCDAP); 1,2-Dilineoyl-3-dimethylammoniumpropan (DLINDAP); Dioleoyloxy-N-[2-(spermincarboxamido)ethyl]-N,N-dimethyl-1-propanaminiumtrifluoroacetat (DOSPA); Dioctadecylamidoglycylspermin (DOGS); 1,2-Dimyristyloxypropyl-3-dimethylhydroxyethylammoniumbromid (DMRIE), 3-Dimethylamino-2-(cholest-5-en-3-beta-oxybutan-4-oxy)-1-(cis,cis-9,12-octadecadienoxy)propan (CLinDMA); 2-[5'-(Cholest-5-en-3-beta-oxy)-3'-oxapentoxy]-3-dimethyl-1-(cis,cis-9',12'-octadecadienoxy)propan (CpLinDMA); N,N-Dimethyl-3,4-dioleyloxybenzylamin (DMOBA); 1,2-N,N'-Dioleylcarbamyl-3-dimethylaminopropan (DOcarbDAP); 1,2-Diacyl-3-trimethylammoniumpropan (TAP); und 1,2-Diacyl-3-dimethylammoniumpropan (DAP) ist.

6. Komplex gemäß einem der vorstehenden Ansprüche, wobei die Nukleinsäure ausgewählt ist aus der Gruppe bestehend aus Small-interfering-RNA (siRNA), ribosomaler RNA (rRNA), Ribonukleinsäure (RNA), Desoxyribonukleinsäure (DNA), komplementärer DNA (cDNA), Aptamer, Messenger-DNA (mRNA), Transfer-RNA (tRNA) und Antisense-Oligodesoxynukleotid (AS-ODN).

7. Verfahren zum Herstellen eines LDL-artigen kationischen Nanopartikels zum Abgeben einer Nukleinsäure, umfassend:
a) Auflösen von Cholesterylester, Triglycerid, Phospholipiden, Cholesterol und eines kationischen Lipids in einem organischen Lösungsmittel;
b) Entfernen des organischen Lösungsmittels aus der Lösung, um einen Lipidfilm zu bilden;
c) Zugeben einer wasserlöslichen Lösung zu dem Lipidfilm, um ihn zu hydratisieren; und
d) Komplexieren des erhaltenen LDL-artigen kationischen Nanopartikels mit der Nukleinsäure.

8. Verfahren zum Herstellen eines LDL-artigen kationischen Nanopartikels zum Abgeben einer Nukleinsäure, umfassend:
a') Auflösen von Cholesterylester, Triglycerid, Phospholipiden, Cholesterol und eines kationischen Lipids;
b') Zugeben von Wasser zu der genannten Lösung und Mischen davon, um ein Gemisch zu erhalten;
c') Rühren des Gemischs, um eine homogene Lösung zu bilden; und
d') Komplexieren des erhaltenen LDL-artigen kationischen Nanopartikels mit der Nukleinsäure.

9. Verfahren gemäß Anspruch 8, wobei das Rühren bei Schritt (c') durch ein Verfahren ausgewählt aus Ultraschallbehandlung, Hochdruckhomogenisierung und die Verwendung eines Membranverflüssigers durchgeführt wird.

10. Verfahren gemäß Anspruch 8 oder 9, ferner umfassend einen Schritt des Entfernens des organischen Lösungsmittels nach Schritt (c').

11. Verfahren gemäß einem der Ansprüche 8-10, wobei Cholesterylester, Triglycerid, Phospholipide, Cholesterol und das kationische Lipid durch Erhitzen gelöst werden.

12. Verfahren gemäß einem der Ansprüche 8-11, wobei Cholesterylester, Triglycerid, Phospholipide, Cholesterol und das kationische Lipid durch Zugeben eines organischen Lösungsmittels gelöst werden.

13. Verfahren gemäß einem der Ansprüche 7-12, wobei das organische Lösungsmittel wenigstens eines ausgewählt aus der Gruppe bestehend aus Chloroform, Methanol und Cyclohexan ist.

14. Verfahren gemäß einem der Ansprüche 7-13, wobei der Komplexierungsschritt (d, d') Inkubieren des LDL-artigen kationischen Nanopartikels in phosphatgepufferter Kochsalzlösung oder entsalztem Wasser in Gegenwart der Nukleinsäure umfasst.

## Revendications

1. Complexe d'un acide nucléique et d'une nanoparticule cationique du type lipoprotéine de faible densité (LDL) destiné à délivrer un acide nucléique comprenant : une partie centrale lipidique contenant un ester de cholestéryle et un triglycéride ; ainsi qu'une partie lipidique de surface cationique contenant du cholestérol, des phospholipides et un lipide cationique, qui forme une surface cationique de la partie centrale lipidique via une interaction hydrophobe, dans lequel le lipide cationique est combiné avec l'acide nucléique via une interaction électrostatique.

2. Complexe selon la revendication 1, dans lequel la nanoparticule cationique comprend de 30 à 60% en poids d'ester de cholestéryle, de 0,1 à 10% en poids de triglycéride, de 5 à 20% en poids de cholestérol, de 5 à 30% en poids de phospholipides et de 10 à 50% en poids d'un lipide cationique.

3. Complexe selon les revendications 1 ou 2, dans lequel un rapport pondéral de la partie centrale lipidique sur la partie lipidique de surface cationique dans la nanoparticule s'échelonne de 30:70 à 70:30.

4. Complexe selon l'une quelconque des revendications précédentes, dans lequel les phospholipides sont au moins un élément choisi dans un groupe constitué par : une dioléoylphosphatidyl éthanolamine (DOPE) ; une palmitoyloléoyl phosphatidyl choline (POPC) ; une phosphatidyl choline d'oeuf (EPC) ; une distéaroylphosphatidyl choline (DSPC) ; une dioléoylphosphatidyl choline (DOPC) ; une dipalmitoylphosphatidyl choline (DPPC) ; un dioléoylphosphatidyl glycérol (DOPG) ; et un dipalmitoylphosphatidyl glycérol (DPPG).

5. Complexe selon l'une quelconque des revendications précédentes, dans lequel le lipide cationique est au moins un élément choisi dans un groupe constitué par :
le 3β-[N-(N',N',N'-triméthylaminoéthane)carbamoyl]cholestérol (TC-cholestérol) ; le 3β-[N-(N',N'-diméthylaminoéthane)carbamoyl]cholestérol (DC-cholestérol) ; le 3β-[N-(N'-monométhylaminoéthane)carbamoyl]cholestérol (MC-cholestérol) ; le 3β-[N-(aminoéthane)carbamoyl]cholestérol (AC-cholestérol) ; le N-(N'-(aminoéthane)carbamoyl tocophérol propanoïque (AC-tocophérol) ; le N-(N-(méthylaminoéthane)carbamoyl tocophérol propanoïque (MC-tocophérol) ; le chlorure de N,N-dioléyl-N,N-diméthylammonium (DODAC) ; le bromure de N,N-distéaryl-N,N-diméthylammonium (DDAB) ; le chlorure de N-(1-(2,3-dioléoyloxy)propyl-N,N,N-triméthylammonium (DOTAP) ; la N,N-diméthyl-(2,3-dioléoyloxy)propylamine (DODMA) ; le chlorure de N-(1-(2,3-dioléyloxy)propyl-N,N,N-triméthylammonium (DOTMA) ; le 1,2-dioléoyl-3-diméthylammonium-propane (DODAP) ; le 1,2-dioléoyl carbamyl-3-diméthylammoniumpropane (DOCDAP) ; le 1,2-dilinéoyl-3-diméthylammoniumpropane (DLINDAP) ; le trifluoroacétate de dioléoyloxy-N-[2-sperminecarboxamido)éthyl]-N,N-diméthyl-1-propanaminium (DOSPA) ; la dioctadécylamidoglycyl spermine (DOGS) ; le bromure de 1,2-dimyristyloxypropyl-3-diméthyl-hydroxyéthyl ammonium (DMRIE), le 3-diméthylamino-2-(cholest-5-en-3-bêta-oxybutane-4-oxy)-1-(cis,cis-9,12-octade cadiénoxy)propane (CLinDMA) ; le 2-[5'-(cholest-5-en-3-bêta-oxy)-3'-oxapentoxy]-3-diméthyl-1-(cis,cis-9',12'-octadécadiénoxy)propane (CpLinDMA) ; la N,N-diméthyl-3,4-dioléyloxybenzylamine (DMOBA) ; le 1,2-N,N'-dioléylcarbamyl-3-diméthylaminopropane (DOcarbDAP) ; le 1,2-diacyl-3-triméthylammonium-propane (TAP) ; et le 1,2-diacyl-3-diméthylammonium-propane (DAP).

6. Complexe selon l'une quelconque des revendications précédentes, dans lequel l'acide nucléique est choisi dans un groupe constitué par un petit ARN interférent (siARN), un ARN ribosomique (ARNr), un acide ribonucléique (ARN), un acide désoxyribonucléique (ADN), un ADN complémentaire (ADNc), un aptamère, un ADN messager (ARNm), un ARN de transfert (ARNt) et un oligodésoxynucléotide antisens (ODN-AS).

7. Procédé destiné à préparer une nanoparticule cationique de type LDL destinée à délivrer un acide nucléique, comprenant les étapes consistant à :
a) dissoudre un ester de cholestéryle, un triglycéride, des phospholipides, du cholestérol et un lipide cationique dans un solvant organique ;
b) ôter le solvant organique de la solution pour former un film lipidique ;
c) ajouter une solution hydrosoluble au film lipidique pour l'hydrater ; et
d) complexer la nanoparticule cationique de type LDL résultante avec l'acide nucléique.

8. Procédé destiné à préparer une nanoparticule cationique de type LDL destinée à délivrer un acide nucléique, comprenant les étapes consistant à :
a') dissoudre un ester de cholestéryle, un triglycéride, des phospholipides, du cholestérol et un lipide cationique ;
b') ajouter de l'eau à la solution ci-dessus et la mélanger pour préparer un mélange ;
c') agiter le mélange pour former une solution homogène ; et
d') complexer la nanoparticule cationique de type LDL résultante avec l'acide nucléique.

9. Procédé selon la revendication 8, dans lequel l'agitation dans l'étape (c') est effectuée par un quelconque processus choisi parmi la sonication, une homogénéisation haute pression et l'utilisation d'un agent fluidifiant membranaire.

10. Procédé, selon les revendications 8 ou 9, comprenant en outre une étape de retrait du solvant organique après l'étape (c').

11. Procédé selon l'une quelconque des revendications 8 à 10, dans lequel les ester de cholestéryle, triglycéride, phospholipides, cholestérol et lipide cationique sont dissous par la chaleur.

12. Procédé selon l'une quelconque des revendications 8 à 11, dans lequel les ester de cholestéryle, triglycéride, phospholipides, cholestérol et lipide cationique sont dissous par un ajout de solvant organique à ceux-ci.

13. Procédé selon l'une quelconque des revendications 7 à 12, dans lequel le solvant organique est au moins un élément choisi dans un groupe constitué par le chloroforme, le méthanol et le cyclohexane.

14. Procédé selon l'une quelconque des revendications 7 à 13, dans lequel l'étape de complexation (d, d'), comprend une incubation de la nanoparticule cationique de type LDL dans une solution saline tamponnée au phosphate ou dans de l'eau dessalée en présence de l'acide nucléique.
